# EUROPEAN PATENT APPLICATION

(11) **EP 3 711 679 A1**
(43) Date of publication of application: **23.09.2020**
(21) Application number: 17935202.6
(22) Date of filing: 22.12.2017
(51) Int. Cl.: A61B 10/00, G06Q 50/22

(54) **PROGRAM FOR USE IN ASSISTING HEALTH MONITORING, HEALTH MONITORING ASSISTANCE METHOD, AND HEALTH MONITORING ASSISTANCE SYSTEM**

(71) Applicant: Unicharm Corporation, Ehime 799-0111 (JP)
(72) Inventor: SUZUKI, Yuya, Kanonji-shi, Kagawa 769-1602 (JP); SASAKI, Toru, Kanonji-shi, Kagawa 769-1602 (JP); ISHIKAWA, Hiroki, Kanonji-shi, Kagawa 769-1602 (JP); TANGE, Akiko, Kanonji-shi, Kagawa 769-1602 (JP); MIMURO, Megumi, Kanonji-shi, Kagawa 769-1602 (JP); IMAGAWA, Takahiro, Kanonji-shi, Kagawa 769-1602 (JP); SHIBAYA, Eiji, Kanonji-shi, Kagawa 769-1602 (JP)
(74) Representative: Dolleymores
(86) International application number: PCT/JP2017/046123
(87) International publication number: WO 2019/123628

(57) **Abstract**

[PROBLEM] It is to provide a program used for health management support, a method for health management support, and a system for health management support, in order for a user of an absorbent article to manage their health.

[SOLUTION] It is a program causing a computer to execute: a transmission process for transmitting, to a server, an image of an absorbent article to which excrement discharged from a vagina of a user is attached; a reception process for receiving health information from the server, the health information being information about a health condition of the user, the health information being obtained on the basis of the image; and a health-information displaying process for displaying on a display a health information screen on the basis of the health information, the health information screen including a first message and a second message, the first message indicating the user's health condition, the second message for improving the health condition.

## Description

### [Technical Field]

The present invention relates to a program used for health management support, a method for health management support, and a system for health management support.

### [Background Art]

Typically, vaginal discharge and menstrual blood discharged from the vagina of a woman changes depending on the health condition. Thus, as illustrated in Patent Document 1, menstrual blood on a sanitary product is extracted, and the health condition of the sanitary product user is analyzed.

### [Citation List]

### [Patent Literature]

[Patent Document 1] U.S. Patent Application Publication 2016/0324506

### [Summary of Invention]

### [Technical Problem]

However, the technology disclosed in Patent Document 1 requires a dedicated system for extracting the menstrual blood on the sanitary product or the like. Thus, it has been difficult for the user to manage their health easily on the basis of information of the menstrual blood or the like.

The present invention was achieved in light of conventional problems such as that described above, and an aspect of the present invention is to provide a program used for health management support, a method for health management support, and a system for health management support.

### [Solution to Problem]

A main aspect of the present invention for achieving the above-described aspect is a program causing a computer to execute:
a transmission process for transmitting, to a server, an image of an absorbent article to which excrement discharged from a vagina of a user is attached;
a reception process for receiving health information from the server,
   the health information being information about a health condition of the user,
   the health information being obtained on the basis of the image; and
a health-information displaying process for displaying on a display a health information screen on the basis of the health information,
   the health information screen including a first message and a second message,
   the first message indicating the user's health condition,
   the second message for improving the health condition.

### [Advantageous Effects of Invention]

According to the present invention, the user can manage their health easily on the basis of information of vaginal discharge.

### [Brief Description of the Drawings]

[FIG. 1] FIG. 1 illustrates a terminal 10 and a server 11.
[FIG. 2] FIG. 2 illustrates a flow of a method for health management support according to an embodiment.
[FIG. 3] FIG. 3 illustrates details of a menses-information process according to the embodiment.
[FIG. 4] FIG. 4 illustrates a message image displayed on a display 23 when an image of a napkin is captured.
[FIG. 5] FIG. 5 illustrates an example of a napkin image 210.
[FIG. 6] FIG. 6 illustrates a message image displayed on the display 23 when menses information is input.
[FIG. 7] FIG. 7 illustrates an example of a determination result of the server 11.
[FIG. 8] FIG. 8 illustrates an example of a health information screen 27.

### [Description of Embodiments]

At least the following matters will become clear with the description of this specification and the attached drawings.
A program causing a computer to execute:
a transmission process for transmitting, to a server, an image of an absorbent article to which excrement discharged from a vagina of a user is attached;
a reception process for receiving health information from the server,
   the health information being information about a health condition of the user,
   the health information being obtained on the basis of the image; and
a health-information displaying process for displaying on a display a health information screen on the basis of the health information,
   the health information screen including a first message and a second message,
   the first message indicating the user's health condition,
   the second message for improving the health condition.

According to such a program, the user is able to know about and improve their health condition on the basis of the information displayed on the health information screen, thereby managing their health easily.

In such a program, it is preferable
that the excrement includes menstrual blood, and
that the program causing the computer to further execute:
   an instruction displaying process for displaying a third message on the display,
   the third message being for prompting to capture an image of the absorbent article when a predetermined number of days has passed since start of a menstruation of the user.

According to such a program, the user is able to capture an image of a napkin when the predetermined number of days has passed since start of a menstruation (e.g., a napkin on the third day since the start of the menstruation), that is a timing when typically the menstrual blood volume becomes stable. Thus, the user is provided with highly accurate information about their health condition.

In such a program, it is preferable
that the third message is displayed for prompting to capture an image of the absorbent article in a morning of a fourth day since the start of the menstruation, and
that the absorbent article is one that has been used overnight on a third day since the start of the menstruation.

According to such a program, at the same timing in each menstruation, the user is able to capture an image of a napkin used. Thus, the user is able to easily know about a monthly change in their health condition.

In such a program, it is preferable
that in the transmission process, user information is further transmitted to the server,
that the user information is information about the user, and
that the health information is obtained on the basis of the image and the user information.

According to such a program, the user is provided with advice based on user's medical-interview information as well as based on the image of the absorbent article to which menstrual blood or the like is attached. This improves the accuracy of the advice for health management.

In such a program, it is preferable
that the excrement includes menstrual blood,
that the program causing the computer to further execute:
   an input-screen displaying process for displaying an input screen on the display,
      the input screen being for inputting menstruation information,
      the menstruation information being information about menstruation when the image of the absorbent article is captured; and
   a menstruation-information reception process for receiving the menstruation information that is input,
that in the transmission process, the menstruation information that has been received is further transmitted to the server, and
that the health information is obtained on the basis of the image and the menstruation information.

According to such a program, the user is provided with advice based on the information of user's menstruation as well as based on the image of the absorbent article to which menstrual blood or the like is attached. This improves the accuracy of the advice for health management.

In such a program, it is preferable
that the excrement includes menstrual blood,
that the program causing the computer to further execute:
   an input-screen displaying process for displaying an input screen on the display,
      the input screen being for inputting menstruation information,
      the menstruation information being information about menstruation when the image of the absorbent article is captured; and
   a menstruation-information reception process for receiving the menstruation information that is input,
that in the transmission process, user information about the user and the menstruation information that has been received are further transmitted to the server, and
that the health information is obtained on the basis of the image, the user information, and the menstruation information.

According to such a program, the user is provided with advice based on user's medical-interview information and user's menstruation information as well as based on the image of the absorbent article to which menstrual blood or the like is attached. This improves the accuracy of the advice for health management.

In such a program, it is preferable
that the menstruation information includes information indicating a number of absorbent articles that have been used by the user before capturing the image of the absorbent article, and
that the input screen includes a fourth message that prompts the user to input the number of absorbent articles.

According to such a program, the number of used absorbent articles, which is in accordance with a menstrual blood volume of the user, is input to the computer as objective information about the menstruation.

In such a program, it is preferable
that the menstruation information includes information indicating color of the menstrual blood attached to the absorbent article, and
that the input screen includes a plurality of colors and a fifth message,
   the fifth message being for making a user select a darkest color of the menstrual blood attached to the absorbent article from the plurality of colors.

According to such a program, the information about the color of the menstrual blood is input to the computer as well as the image of the absorbent article to which menstrual blood or the like is attached. This makes it possible for the user to be provided with high-quality advice even if there is a problem in the method for capturing the image of the absorbent article to which menstrual blood is attached, the image capturing environment, the quality of the image of the absorbent article, and the like.

In such a program, it is preferable
that the menstruation information includes information indicating whether there is any clotted matter in the excrement, and
that the input screen includes a sixth message that prompts the user to input whether there is any clotted matter in the excrement.

According to such a program, the information indicating whether there is any clotted matter in the excrement is input to the computer as well as the image of the absorbent article to which menstrual blood or the like is attached. This makes it possible for the user to be provided with high-quality advice even if it is difficult to determine in the image of the absorbent article whether there is any clotted matter in the excrement.

In such a program, it is preferable that
the program causing the computer to further execute a field-instruction displaying process for displaying a field instruction image on the display,
the field instruction image being an image for prompting to capture an image of a to-be-imaged absorbent article so that the image includes a portion of the to-be-imaged absorbent article where no menstrual blood is attached.

According to such a program, the user is able to capture the entire image of menstrual blood attached to the absorbent article. This makes it possible to provide the user with higher-quality advice, compared with a case, for example, where an image of only a portion of the menstrual blood attached to the absorbent article is captured.

In such a program, it is preferable that
the first message is a message about stress or hormone balance.

According to such a program, the user is able to obtain knowledge about their stress level or hormone imbalance.

In such a program, it is preferable that
the second message is a message about a nutrient that the user should take.

According to such a program, the user is able to improve their health condition by having appropriate meals in accordance with the content of the message.

In such a program, it is preferable that
the health information is information about stress or hormone balance,
the program causing the computer to further execute
   a generating process for generating information indicating the first message and the second message on the basis of the health information.

According to such a program, the computer generates the messages, and thus, the server processes and burden can be reduced.

A method for health management support, including:
a transmission step in which a computer transmits to a server an image of an absorbent article to which excrement discharged from a vagina of a user is attached;
a reception step in which the computer receives health information from the server,
   the health information being information about a health condition of the user,
   the health information being obtained on the basis of information transmitted from the computer; and
a health-information displaying step in which the computer displays on a display a health information screen on the basis of the health information,
   the health information screen including a first message and a second message,
   the first message indicating the user's health condition,
   the second message for improving the health condition.

According to such a method, the user is able to know about and improve their health condition on the basis of the information displayed on the health information screen, thereby managing their health easily.

A computer system including:
a transmission module for transmitting, to a server, an image of an absorbent article to which excrement discharged from a vagina of a user is attached;
a reception module for receiving health information from the server,
   the health information being information about a health condition of the user,
   the health information being obtained on the basis of information transmitted from the computer; and
a health-information displaying module for displaying on a display a health information screen on the basis of the health information,
   the health information screen including a first message and a second message,
   the first message indicating the user's health condition,
   the second message for improving the health condition.

According to such a computer system, the user is able to know about and improve their health condition on the basis of the information displayed on the health information screen, thereby managing their health easily.

### Embodiment

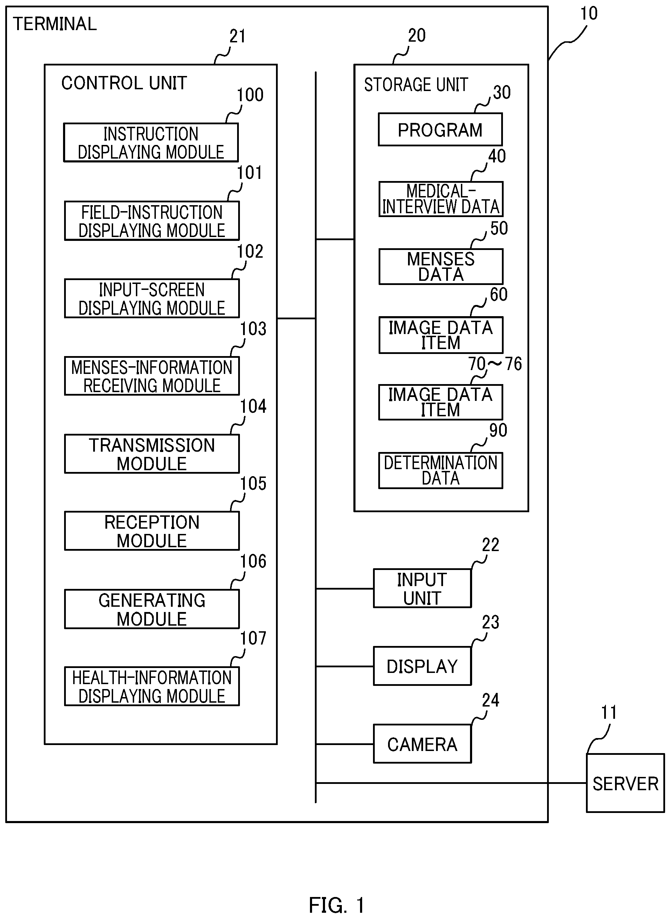

FIG. 1 illustrates a terminal 10 and a server 11. The terminal 10 is a smartphone or tablet, and implements various functions. In addition, by performing various processings, the terminal 10 displays, on a display 23 of the terminal 10, advice about the health condition of a user of the terminal 10 (hereinafter simply referred to as "user") or the like.

The server 11 is an apparatus to which the terminal 10 is connected via a network and transmits or receives various types of information to or from the terminal 10.

The terminal 10 (computer) includes a storage unit 20, a control unit 21, an input unit 22, the display 23, and a camera 24.

The storage unit 20 stores a program 30 executed by a processor (not illustrated) of the terminal 10, medical-interview data 40, menses data 50, an image data item 60, image data items 70 to 76, and determination data 90. The program 30 is used when supporting the user's health management.

The medical-interview data 40 (user information) is data indicating "medical-interview information" of the user and includes information about a profile, lifestyle, and menses and condition. Herein, the information about a profile is information about, for example, the user's age, height, weight, and blood type, and whether the user has any children. The information about lifestyle is information about, for example, having balanced meals, good quality sleep, and a stress relief method. The information about menses and condition is information about, for example, a menstrual cycle, the number of menstrual days, menstrual blood volume, period pain, and premenstrual symptoms.

The menses data 50 (menstruation information) is data indicating "menses information" about the period (menstruation) of the user.

The image data item 60 is data indicating an image of a sanitary napkin (hereinafter simply referred to as "napkin (absorbent article)") to which the menstrual blood (excrement discharged from a vagina) of the user is attached.

Each of the image data items 70 to 76 is data indicating an image (e.g., message image or illustration image) to be displayed as appropriate on the display 23 of the terminal 10 when the program 30 is executed.

The determination data 90 (health information) is data about the user's health condition obtained by processings of the server 11. Note that the server 11 executes a predetermined program to determine the user's health condition on the basis of the medical-interview data 40, the menses data 50, and the image data item 60. Herein, a storage unit (not illustrated) of the server 11 stores a large number of information items indicating determination results of the user's health condition that a physician has determined on the basis of the "image data item 60", the "menses information", and the "medical-interview information" (that is, previously accumulated information items of the user). The server 11 performs pattern matching or the like by using the accumulated information items, generating the "determination data 90" indicating the user's health condition.

Although the determination data 90 herein is generated by the server 11 performing processings, the determination data 90 is not limited to this. For example, upon receiving the medical-interview data 40, the menses data 50, and the image data item 60, the server 11 displays, on a monitor (not illustrated), the user's "medical-interview information" and "menses information" and a napkin image 210 on the basis of the above data items. On the basis of these displayed information items, a physician may determine the user's health condition (stress or hormone balance) and input the determination data 90 to the server 11.

The control unit 21 is a functional block which is implemented in the terminal 10 by a processor (not illustrated) provided in the terminal 10 executing the program 30. The control unit 21 controls the entire terminal 10 and includes a plurality of blocks that execute various processings. Note that details of functional blocks implemented by the control unit 21 will be described later.

An operation result of the terminal 10 by the user is input to the input unit 22. The input unit 22 is implemented as a tap operation on the touch-panel display 23 of the terminal 10.

The display 23 displays various types of information on the basis of a command from the control unit 21 or an operation result by the user. The camera 24 obtains image data by capturing an image.

### Functional Blocks Implemented in Control Unit 21

The control unit 21 includes an instruction displaying module 100, a field-instruction displaying module 101, an input-screen displaying module 102, a menses-information receiving module 103, a transmission module 104, a reception module 105, a generating module 106, and a health-information displaying module 107.

The instruction displaying module 100 performs a processing for displaying, on the display 23, a message image (described later) for prompting the user to capture an image of a napkin that is used overnight on the third day; the third day of a menstruation is a day when two days (predetermined number of days) have lapsed from the start of a period (start of menstruation) and typically the menstrual blood volume becomes stable.

The field-instruction displaying module 101 performs a processing for displaying, on the display 23, a field-instruction image (described later) for capturing an image of a napkin that is an image capturing target, so as to instruct for the user to include a portion of the napkin where no menstrual blood is attached (margin on the surface of the napkin).

The input-screen displaying module 102 performs a processing for displaying, on the display 23, an input screen (described later) for inputting the user's "menses information". The menses-information receiving module 103 performs a processing for receiving the "menses information" that has been input.

The transmission module 104 transmits, to the server 11, the medical-interview data 40, the menses data 50, and the image data item 60, which are stored in the storage unit 20. The reception module 105 receives the determination data 90 from the server 11 and stores the determination data 90 in the storage unit 20.

The generating module 106 generates various message images (described later) about the user's health condition, on the basis of the determination data 90 about the user's health condition.

The health-information displaying module 107 performs a processing for displaying, on the display 23, a message image related to the health condition generated by the generating module 106.

### Method for Health Management Support

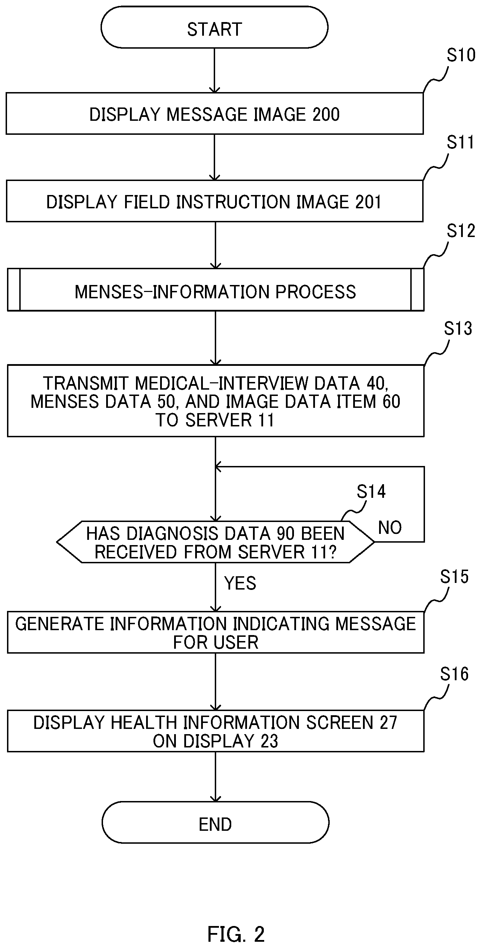

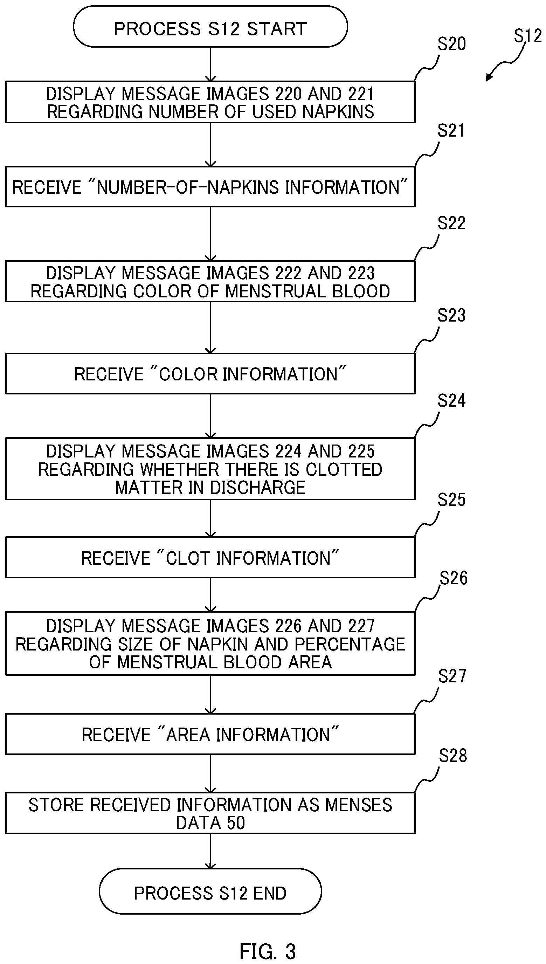

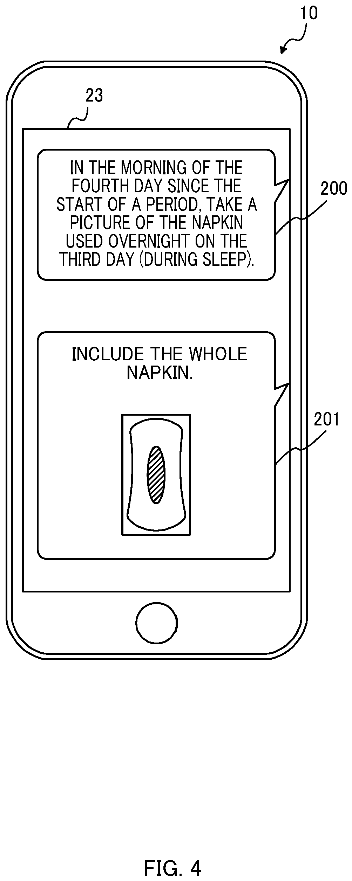

Now, a flow of a method for health management support will be described with reference to FIG. 2 and FIG. 3. Herein, the program 30 has been started as a result of an operation on the terminal 10 by the user. When the user operates a main screen (not illustrated) displayed on the display 23, the instruction displaying module 100 displays an image illustrated in FIG. 4 on the display 23.

Specifically, the instruction displaying module 100 displays, on the display 23 and on the basis of the image data item 70, a message image 200 (third message) for capturing an image of a napkin that is used overnight on the third day (S10: instruction displaying process). Note that the message image 200 indicates "In the morning of the fourth day since the start of a period, take a picture of the napkin used overnight on the third day (during sleep)". Typically, the menstrual blood volume becomes stable at night on the third day since the start of a period. Thus, the user captures an image of the used napkin in the morning of the fourth day, thereby obtaining an image at a time suitable for determination. In addition, the user captures an image of a napkin at the same time (in the morning of the fourth day) in each month, and thus, the user can easily know a change in their health condition.

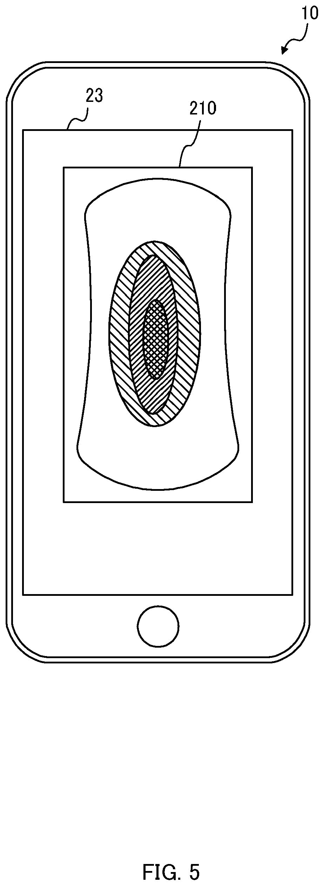

Subsequently, the field-instruction displaying module 101 displays, on the display 23 and on the basis of the image data item 71, a field instruction image 201 as an instruction of a field for capturing an image of a napkin (S11: field-instruction displaying process). Note that the field instruction image 201 includes the message "Include the whole napkin" and a reference image of the image capturing target. In the morning of the fourth day, when the user operates the terminal 10 to capture an image of a napkin used during sleep on the third day, an image of the napkin to which the menstrual blood is attached (hereinafter, the napkin image 210) illustrated in FIG. 5 is stored as the image data item 60 in the storage unit 20.

Note that, for example, if the user captures an image of only part of the menstrual blood attached to the napkin, the amount of information about the color and volume of the menstrual blood in the image, which is important for determination, is small. However, in this embodiment, since the field instruction image 201 is displayed, the user can capture an image suitable for determination.

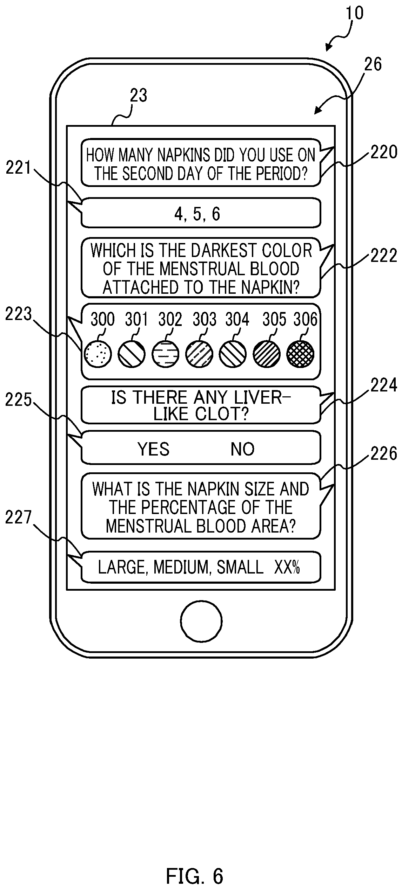

FIG. 3 is a flowchart illustrating an example of a process performed in a menses-information process (S12), and FIG. 6 illustrates an example of an input screen 26 displayed on the display 23.

First, as illustrated in FIG. 6, the input-screen displaying module 102 displays, on the display 23 and on the basis of the image data item 72, a message image 220 (fourth message) that prompts the user to input the number of napkins used since the start to the second day of a period and an image 221 of a plurality of choices corresponding to the number of used napkins (S20: input-screen displaying process). The message image 220 indicates "How many napkins did you use on the second day of the period?", and the image 221 indicates "4, 5, 6".

If the user selects "5" by tapping the display 23, for example, the menses-information receiving module 103 receives the selection result as "number-of-napkins information" (S21: menstruation-information reception process).

The input-screen displaying module 102 displays, on the display 23 and on the basis of the image data item 73, a message image 222 (fifth message) for making a user select the darkest color of the menstrual blood attached to the napkin and an image 223 indicating a plurality of colors (S22: input-screen displaying process). Note that the message image 222 indicates "Which is the darkest color of the menstrual blood attached to the napkin?", and the image 223 indicates seven colors 300 to 306. In the image 223 herein, the color becomes darker toward the right side (the color 306 side). Thus, among the colors 300 to 306, the color 300 is the lightest, and the color 306 is the darkest.

When the user selects the color 306 by tapping the display 23, for example, the menses-information receiving module 103 receives the selection result as "color information" (S23: menstruation-information reception process).

The input-screen displaying module 102 displays, on the display 23 and on the basis of the image data item 74, a message image 224 (sixth message) that prompts the user to input whether there is any clotted matter in the excrement and a message image 225 indicating choices (S24: input-screen displaying process). Note that the message image 224 indicates "Is there any liver-like clot?", and the message image 225 indicates "Yes, No".

If the user selects "Yes" by tapping the display 23, for example, the menses-information receiving module 103 receives the selection result as "clot information" (S25: menstruation-information reception process).

The input-screen displaying module 102 displays, on the display 23 and on the basis of the image data item 75, a message image 226 that prompts the user to input the size of the napkin and a state of dispersion (area) of the menstrual blood. Also, the input-screen displaying module 102 displays a message image 227 that prompts the user to input (select) such information (S26). Note that the message image 226 indicates "What is the napkin size and the percentage of the menstrual blood area?", and the message image 227 indicates "Large, Medium, Small, xx%".

If the user selects "Medium" and inputs "50%" by tapping the display 23, for example, the menses-information receiving module 103 receives the input results as "area information" (S27). Note that the message image 226 may indicate choices "Large, Medium, Small, less than 30% of the whole napkin area, 30% to less than 50%, 50% to less than 80%, more than 80%", for example. Alternatively, as the "area information", for example, the menses-information receiving module 103 may receive the menstrual blood area traced in the napkin image 210 by the user with a finger. The napkin size is selected herein, but without limitation to this, for example, the usage or size (e.g., daytime xx cm or nighttime xx cm) of the napkin may be selected or input.

The menses-information receiving module 103 stores the received "number-of-napkins information", "color information", "clot information", and "area information" as the menses data 50 in the storage unit 20.

When step S12 ends, the transmission module 104 transmits to the server 11 the medical-interview data 40, the menses data 50, and the image data item 60, which have been stored in the storage unit 20 (S13: transmission process). Upon receiving the medical-interview data 40, the menses data 50, and the image data item 60, the server 11 displays, on a monitor (not illustrated), the user's "medical-interview information" and "menses information" and the napkin image 210 on the basis of the above data items. On the basis of these displayed information items, the server 11 determines the user's health condition (stress or hormone balance).

Herein, the server 11 refers to the "number-of-napkins information", the "color information", the "clot information", and the "area information" included in the "menses information" for determination. The "number-of-napkins information" is information indicating the menstrual blood volume from the start to the second day of a period of the user as a quantity of the "number of napkins". And the "area information" is also information indicating the menstrual blood volume of the user as a quantity. Thus, if menorrhagia is determined on the basis of these information items, it is determined that the user may possibly have a symptom of anemia or the like. Note that the server 11 can determine the symptom with high accuracy because when running the predetermined program referred to are the "number of napkins" used and the actual area in which the menstrual blood is attached. In addition, when capturing the image of the napkin to which the menstrual blood is attached, the color in the image may change depending on uneven light on the napkin, reflection of light, characteristics of the camera 24, and the like. Thus, typically, it is difficult to accurately determine the color of the menstrual blood attached to the napkin. However, in this embodiment, the server 11 can refer to the "color information" about the color of the menstrual blood selected by the user by visual inspection. Thus, the amount of information that is input to the server 11 is increased, and the color of the menstrual blood can be determined more accurately. In addition, depending on the volume or color of the menstrual blood attached to the napkin, it is in some cases difficult to determine whether a liver-like clot is included in the image. In this embodiment, the server 11 can refer to the "clot information" indicating whether the liver-like clot is included, and thereby the determination accuracy is increased.

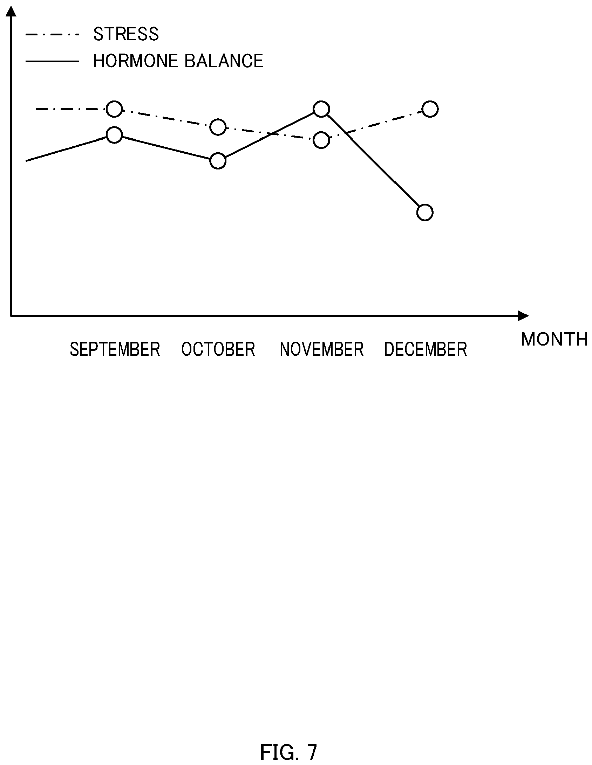

FIG. 7 illustrates an example of the "determination result" of the server 11. The server 11 quantifies "stress" and "hormone balance" and records them as the "determination result" in a storage unit (not illustrated) of the server 11. Note that the chained line in FIG. 7 represents "stress" of the user, and the solid line represents "hormone balance" of the user. Herein, as the user's stress is smaller, the value of "stress" is increased, and as the user's hormone balance is better, the value of "hormone balance" is increased. The server 11 herein, for example, updates "stress" and "hormone balance" in December.

Subsequently, the server 11 transmits the "determination result" about the user's health condition as the determination data 90 to the terminal 10. The reception module 105 of the terminal 10 determines whether the determination data 90 has been received from the server 11 (S14). When the determination data 90 is received from the server 11 (S14: Yes, reception process), the generating module 106 generates information (the image data item 76) indicating a message for the user, on the basis of the determination data 90 (S15: generating process).

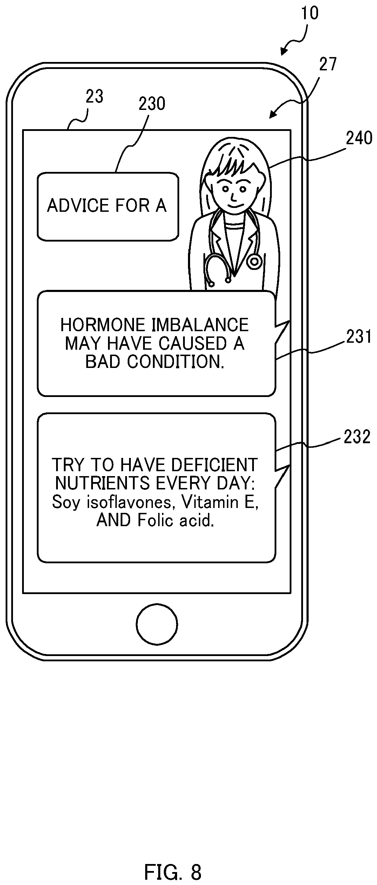

In addition, on the basis of the information generated in step S15, the health-information displaying module 107 displays, on the display 23, a health information screen 27 illustrated in FIG. 8 (S16: health-information displaying process). Herein, the health information screen 27 includes message images 230 to 232 and a cartoon character image 240 of a physician. The message image 230 indicates "advice for A"; who is the user. The message image 231 (first message) indicates the user's health condition, indicating "Hormone imbalance may have caused a bad condition". The message image 232 (second message) is information for improving the user's health condition, indicating "Try to have deficient nutrients every day: Soy isoflavones, Vitamin E, and Folic acid". Thus, by referring to the health information screen 27, the user is able to know their health condition. Furthermore, the user is able to know the nutrients to have by referring to the health information screen 27, thereby managing their health.

Note that the messages illustrated in FIG. 8 are examples, and the messages are not limited to these. For example, if menorrhagia is determined from the "menstruation information" and the napkin image, the message image 231 indicating the health condition may be "You have menorrhagia and may be anemic". In such a case, the message image 232 may be "Supplement with iron".

In addition, for example, the message image 231 may be a message about stress, indicating "You may be highly stressed". In such a case, the message image 232 may be advice for relaxing the user and relieving the stress, such as "Take a slow deep breath", "Hot yoga is recommended", or "Listen to your favorite music". In this manner, the message image 231 includes the user's health condition (e.g., stress, hormone, or condition), and the message image 232 includes advice for improving the user's health condition (e.g., meal, condition management, exercise, or method for relieving stress).

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof. For example, modification which will be described below is possible.

In this embodiment, an image of a napkin to which menstrual blood is attached is captured, and the napkin image 210 is transmitted to the server 11, but without limitation to this. For example, it is acceptable that, while a user is not on a period, an image of a napkin to which "vaginal discharge", "occult blood", or the like excrement discharged from the vagina of the user of the napkin is captured. In such a case, the image of the napkin to which "vaginal discharge" is attached is transmitted to the server 11, and thus, on the basis of information of "vaginal discharge" or the like, the server 11 can determine stress of the user or the like.

In addition, in step S15, the generating module 106 generates information indicating a message to the user on the basis of the determination data 90, but without limitation to this. For example, the server 11 (or a physician) may create the message for the user. In such a case, the determination data 90 includes the message images 231 and 232 for the user. Thus, on the basis of the determination data 90, the health-information displaying module 107 may display the health information screen 27 on the display 23.

In addition, on the input screen 26 displayed is the message image 224 that prompts the user to input whether there is any clotted matter in the excrement, but without limitation to this. For example, on the basis of the napkin image 210, the server 11 may determine whether there is any clotted matter in the excrement, by using an image processing program.

In addition, on the input screen 26 displayed is the message image 227 for designating the napkin size and the percentage of the menstrual blood area, but without limitation to this. For example, on the basis of the napkin image 210, the server 11 may determine the percentage of the menstrual blood (area relative to the whole area), by using an image processing program.

In addition, as in step S12, the message images are displayed sequentially on the input screen 26, but without limitation to this. For example, all of the message images 220 to 227 may be displayed on the display 23 at the same time.

In addition, information about the number of napkins used can be selected by using choices on the input screen 26, but without limitation to this. For example, the number of napkins used may be directly input on the display 23.

In addition, although the image capturing target in this embodiment is the "napkin" used by the user, other absorbent articles, such as a "liner" or "diaper" may also be used, for example.

The terminal 10 transmits three data items, which are the medical-interview data 40, the menses data 50, and the image data item 60, to the server 11, but without limitation to this. For example, at least the image data item 60 may be transmitted. In such a case, the server 11 determines the user's health condition on the basis of the image data item 60.

Furthermore, the program 30 is stored in the storage unit 20 of the terminal 10. Alternatively, for example, the program 30 may be stored on a medium (DVD: digital versatile disc) that stores data or the like and may be executed by a processor (not illustrated) of the terminal 10.

In addition, a program may be supplied to a computer by using a non-transitory computer readable medium with an executable program thereon. Note that examples of the non-transitory computer readable medium include a magnetic recording medium (e.g., flexible disk, magnetic tape, or hard disk drive), a CD-ROM (read only memory), and the like.

### [Reference Signs List]

- 10: terminal
- 11: server
- 20: storage unit
- 21: control unit
- 22: input unit
- 23: display
- 24: camera
- 26: input screen
- 27: health information screen
- 30: program
- 40: medical-interview data
- 50: menses data
- 60, 70 to 76: image data item
- 90: determination data
- 100: instruction displaying module
- 101: field-instruction displaying module
- 102: input-screen displaying module
- 103: menses-information receiving module
- 104: transmission module
- 105: reception module
- 106: generating module
- 107: health-information displaying module
- 200, 220, 222, 224 to 227, 230 to 232: message image
- 221, 223: image
- 201: field instruction image
- 210: napkin image
- 240: cartoon character image

## Claims

1. A program causing a computer to execute:
a transmission process for transmitting, to a server, an image of an absorbent article to which excrement discharged from a vagina of a user is attached;
a reception process for receiving health information from the server,
the health information being information about a health condition of the user,
the health information being obtained on the basis of the image; and
a health-information displaying process for displaying on a display a health information screen on the basis of the health information,
the health information screen including a first message and a second message,
the first message indicating the user's health condition,
the second message for improving the health condition.

2. The program according to Claim 1, wherein
the excrement includes menstrual blood, and
the program causing the computer to further execute:
an instruction displaying process for displaying a third message on the display,
the third message being for prompting to capture an image of the absorbent article when a predetermined number of days has passed since start of a menstruation of the user.

3. The program according to Claim 2, wherein
the third message is displayed for prompting to capture an image of the absorbent article in a morning of a fourth day since the start of the menstruation, and
the absorbent article is one that has been used overnight on a third day since the start of the menstruation.

4. The program according to any one of Claims 1 to 3, wherein
in the transmission process, user information is further transmitted to the server,
the user information is information about the user, and
the health information is obtained on the basis of the image and the user information.

5. The program according to any one of Claims 1 to 3, wherein
the excrement includes menstrual blood,
the program causing the computer to further execute:
an input-screen displaying process for displaying an input screen on the display,
the input screen being for inputting menstruation information,
the menstruation information being information about menstruation when the image of the absorbent article is captured; and
a menstruation-information reception process for receiving the menstruation information that is input,
in the transmission process, the menstruation information that has been received is further transmitted to the server, and
the health information is obtained on the basis of the image and the menstruation information.

6. The program according to any one of Claims 1 to 3, wherein
the excrement includes menstrual blood,
the program causing the computer to further execute:
an input-screen displaying process for displaying an input screen on the display,
the input screen being for inputting menstruation information,
the menstruation information being information about menstruation when the image of the absorbent article is captured; and
a menstruation-information reception process for receiving the menstruation information that is input,
in the transmission process, user information about the user and the menstruation information that has been received are further transmitted to the server, and
the health information is obtained on the basis of the image, the user information, and the menstruation information.

7. The program according to Claim 5 or 6, wherein
the menstruation information includes information indicating a number of absorbent articles that have been used by the user before capturing the image of the absorbent article, and
the input screen includes a fourth message that prompts the user to input the number of absorbent articles.

8. The program according to any one of Claims 5 to 7, wherein
the menstruation information includes information indicating color of the menstrual blood attached to the absorbent article, and
the input screen includes a plurality of colors and a fifth message,
the fifth message being for making a user select a darkest color of the menstrual blood attached to the absorbent article from the plurality of colors.

9. The program according to any one of Claims 5 to 8, wherein
the menstruation information includes information indicating whether there is any clotted matter in the excrement, and
the input screen includes a sixth message that prompts the user to input whether there is any clotted matter in the excrement.

10. The program according to any one of Claims 1 to 9, wherein
the program causing the computer to further execute a field-instruction displaying process for displaying a field instruction image on the display,
the field instruction image being an image for prompting to capture an image of a to-be-imaged absorbent article so that the image includes a portion of the to-be-imaged absorbent article where no menstrual blood is attached.

11. The program according to any one of Claims 1 to 10, wherein
the first message is a message about stress or hormone balance.

12. The program according to any one of Claims 1 to 11, wherein
the second message is a message about a nutrient that the user should take.

13. The program according to any one of Claims 1 to 12, wherein
the health information is information about stress or hormone balance,
the program causing the computer to further execute
a generating process for generating information indicating the first message and the second message on the basis of the health information.

14. A method for health management support, comprising:
a transmission step in which a computer transmits to a server an image of an absorbent article to which excrement discharged from a vagina of a user is attached;
a reception step in which the computer receives health information from the server,
the health information being information about a health condition of the user,
the health information being obtained on the basis of information transmitted from the computer; and
a health-information displaying step in which the computer displays on a display a health information screen on the basis of the health information,
the health information screen including a first message and a second message,
the first message indicating the user's health condition,
the second message for improving the health condition.

15. A computer system comprising:
a transmission module for transmitting, to a server, an image of an absorbent article to which excrement discharged from a vagina of a user is attached;
a reception module for receiving health information from the server,
the health information being information about a health condition of the user,
the health information being obtained on the basis of information transmitted from the computer; and
a health-information displaying module for displaying on a display a health information screen on the basis of the health information,
the health information screen including a first message and a second message,
the first message indicating the user's health condition,
the second message for improving the health condition.
